# EUROPÄISCHE PATENTSCHRIFT

(11) **EP 2 174 680 B1**
(45) Veröffentlichungstag und Bekanntmachung des Hinweises auf die Patenterteilung: **05.10.2011**
(21) Anmeldenummer: 08017529.2
(22) Anmeldetag: 07.10.2008
(51) Int. Cl.: A61M 5/158, A61B 5/00, A61B 17/34

(54) **Insertionsvorrichtung**
Insertion device
Dispositif d'insertion

(43) Veröffentlichungstag der Anmeldung: 14.04.2010
(73) Patentinhaber: Roche Diagnostics GmbH, 68305 Mannheim (DE); F.Hoffmann-La Roche AG, 4070 Basel (CH)
(72) Erfinder: Frank Deck, 67150 Niederkirchen (DE); Hörauf, Christian, 68723 Oftersheim (DE); Keil, Michael, 67071 Ludwigshafen (DE); Konya, Ahmet, 67165 Waldsee (DE); Weiss, Thomas, 68307 Mannheim (DE); Kube, Oliver, 67547 Worms (DE); Ebert, Karl-Peter, 64407 Fränkisch-Crumbach (DE)
(74) Vertreter: Twelmeier Mommer & Partner

(56) Entgegenhaltungen:
- EP-A- 1 829 578
- WO-A-2004/098684
- US-A1- 2002 123 740
- US-A1- 2008 208 139

## Beschreibung

Die Erfindung betrifft eine Insertionsvorrichtung mit den im Oberbegriff des Anspruchs 1 angegebenen Merkmalen. Derartige Insertionsvorrichtungen sind aus der WO 2004/0986684, der EP 1 829 578 und der US 2002/123740 bekannt.

Um Sensoren zur Messung von Analytkonzentrationen in vivo, beispielsweise der Glukosekonzentrationen, in Körpergewebe eines Patienten zu insertieren, beispielsweise im Unterhautfettgewebe, werden Insertionsvorrichtungen verwendet, die mit einem Antriebsmechanismus eine Stichbewegung einer Insertionsnadel bewirken. Hierfür gebräuchliche Insertionsnadeln sind als Hohlnadeln oder V-förmige Rinnen ausgebildet, in denen ein Sensor liegt. Der Sensor kann beispielsweise als ein Elektrodensystem für elektrochemische Messungen ausgebildet sein oder einen mikrofluidischen Katheter zum Ein- und Ausleiten einer Perfusionsflüssigkeit umfassen. Nach einem Einstich wird die Insertionsnadel aus dem Körpergewebe herausgezogen, wobei der Sensor in der erzeugten Stichwunde verbleibt.

Eine weitere Anwendung von Insertionsvorrichtungen ist beispielsweise die Applikation von Kathetern, beispielsweise zur Infusion von Insulin oder anderen Wirkstoffen.

Bei einfachen Insertionsvorrichtungen setzt ein Antriebsmechanismus eine Antriebsbewegung eines Betätigungselements in eine lineare Stichbewegung der Insertionsnadel um. Die für einen Stich erforderliche Kraft muss ein Benutzer durch eine entsprechende Antriebsbewegung des Betätigungselements also während der Insertion selbst aufbringen. Dies macht es vielen Benutzern mühsam, derartige Insertionsvorrichtungen bei sich selbst anzuwenden, beispielsweise um sich einen Sensor im Unterhautfettgewebe des Bauchs zu insertieren. Insbesondere Menschen, deren Beweglichkeit durch Alter oder Krankheit eingeschränkt ist, fällt es schwer, eine Insertionsvorrichtung im richtigen Winkel an ihren Körper zu halten und dabei die für einen Stich erforderliche Kraft aufzubringen. Das Aufbringen von Kraft erschwert es nämlich, die Insertionsvorrichtung beim Betätigen ruhig zu halten und nicht zu verkippen. Eine zittrige Hand beim Stechen oder ein Kippen der Insertionsvorrichtung führen zu schmerzhaften Querbewegungen der Insertionsnadel und im Extremfall zu einem gescheiterten Insertionsversuch. Insbesondere können Querbewegungen beim Einstich dazu führen, dass der insertierte Sensor durch umgebendes Körpergewebe später mechanischen Spannungen ausgesetzt ist, die den Sensor belasten und sogar verbiegen können. Zudem wird das Körpergewebe dabei ständig gereizt, so dass verstärkt Entzündungen und Abstoßungsreaktionen auftreten, die Messungen des Sensors beeinträchtigen.

Diese Nachteile lassen sich durch aufwendigere Insertionsvorrichtungen mit federgetriebenen Antriebsmechanismen weitgehend beheben. Bei derartigen Insertionsvorrichtungen wird die für eine Stichbewegung erforderliche Energie von einer Antriebsfeder oder einem anderen Energiespeicher geliefert. Zur Insertion muss ein Benutzer eine derartige Insertionsvorrichtung lediglich an eine geeignete Körperstelle ansetzen und einen Stich auslösen, beispielsweise durch Druck auf ein Auslöseelement. Der Kraftaufwand hierfür ist minimal, so dass es selbst Personen mit eingeschränkter Beweglichkeit leicht fällt, die Insertionsvorrichtung beim Insertionsvorgang ruhig zu halten.

Aufgabe der Erfindung ist es, einen Weg aufzuzeigen, wie mit geringerem Aufwand eine einfach zu handhabende Insertionsvorrichtung zur Verfügung gestellt werden kann.

Diese Aufgabe wird durch eine Insertionsvorrichtung mit den im Anspruch 1 angegebenen Merkmalen gelöst. Vorteilhafte Weiterbildungen der Erfindung sind Gegenstand der Unteransprüche.

Bei einer erfindungsgemäßen Insertionsvorrichtung setzt der Antriebsmechanismus eine quer oder entgegengesetzt zur Stichrichtung verlaufende Antriebsbewegung von einem oder mehreren Betätigungselementen in eine Stichbewegung des Insertionsnadelhalters um. Obwohl ein Benutzer die Kraft für eine Stichbewegung durch manuelles Einwirken auf das Betätigungselement selbst aufbringen muss, lässt sich eine erfindungsgemäße Insertionsvorrichtung sehr leicht handhaben, indem die Antriebsbewegung des Betätigungselements quer zur Stichrichtung verläuft. Eine Bewegung quer zur Stichrichtung lässt sich nämlich aus ergonomischen Gründen wesentlich leichter durchführen als eine Bewegung in Stichrichtung. Ähnliches gilt für eine Bewegung entgegengesetzt zur Stichrichtung. Selbst Personen mit eingeschränkter Beweglichkeit können sich deshalb mit einer erfindungsgemäßen Insertionsvorrichtung mit ruhiger Hand einen Sensor oder einen Infusionskatheter einsetzen, beispielsweise in den Oberarm oder das Unterhautfettgewebe des Bauchs.

Eine erfindungsgemäße Insertionsvorrichtung hat zudem einen wichtigen psychologischen Vorteil. Da die für eine Stichbewegung erforderliche Kraft quer oder entgegengesetzt zur Stichrichtung aufgebracht wird, hat ein Benutzer nicht das Gefühl, diese Kraft gegen sich selbst zu richten. Selbst wenn die Stichbewegung während der Antriebsbewegung erfolgt, muss zum Betätigen der Insertionsvorrichtung allenfalls eine kleine psychologische Hemmschwelle überwunden werden. Die bei jedem Menschen mehr oder weniger stark ausgeprägte Hemmschwelle dagegen, wissentlich einen spitzen Gegenstand mit einer erhöhten Kraft in Richtung des eigenen Körpers zu treiben, entfällt bei einer erfindungsgemäßen Insertionsvorrichtung.

Ein weiterer Vorteil der erfindungsgemäßen Lösung, die Antriebsbewegung des Betätigungselements quer zur Stichrichtung auszuführen, liegt darin, dass die Insertionsvorrichtung selbst und nicht der eigene Körper als Widerlager der Betätigungskraft wirkt. Dies erleichtert es einem Benutzer, das Körperteil, in das mit der Insertionsnadel eingestochen werden soll, zu entspannen, die Insertionsvorrichtung ruhiger zu halten und trägt auf diese Weise zu einer schmerzärmeren Insertion mit weniger mechanischen Spannungen bei.

Eine erfindungsgemäße Insertionsvorrichtung kann so konstruiert sein, dass die quer zur Stichrichtung verlaufende Antriebsbewegung des Betätigungselements senkrecht zur Stichrichtung erfolgt. Zwingend erforderlich ist dies jedoch nicht. Auch eine Antriebsbewegung, die in einer anderen nicht zur Stichrichtung parallelen Richtung erfolgt, ist quer zur Stichrichtung und ermöglicht es, die vorstehend beschriebenen Vorteile zu nutzen. Insbesondere ist es nicht unbedingt erforderlich, dass die Antriebsbewegung des Betätigungselements eine lineare Bewegung ist. Beispielsweise kann die Antriebsbewegung des Betätigungselements auch eine Schwenkbewegung sein. Je kleiner nämlich der Winkel ist, der bei einer Schwenkbewegung überstrichen wird und je größer der Radius der Schwenkbewegung ist, desto geringer wird ergonomisch der Unterschied zu einer linearen Bewegung.

Für eine erfindungsgemäße Insertionsvorrichtung wird vorteilhaft weder eine Feder noch ein anderer Energiespeicher benötigt. Der Antriebsmechanismus einer erfindungsgemäßen Insertionsvorrichtung kann deshalb energiespeicherfrei und folglich kostengünstig sein. Eine erfindungsgemäße Insertionsvorrichtung kann deshalb auch als ein Wegwerfartikel ausgebildet sein, der nach einmaligem Gebrauch entsorgt wird. Eine Sicherung gegen eine unerwünschte Mehrfachverwendung kann erreicht werden, indem sich der Antriebsmechanismus nach Gebrauch verriegelt, beispielsweise indem das Betätigungselement am Ende der Antriebsbewegung einrastet.

Sofern eine besonders schnelle Stichbewegung der Insertionsnadel gewünscht ist, kann dies erreicht werden, indem die Hand des Benutzers als Energiespeicher genutzt wird. Eine Möglichkeit hierzu ist es, eine Bewegung des Betätigungselements durch eine Sperre zu blockieren, die erst bei Erreichen einer vorgegeben Kraft überwunden wird, beispielsweise indem ein Sperrelement bricht oder durch elastische Verformung überwunden wird. Das plötzliche Überwinden einer solchen Sperre führt dazu, dass der Benutzer das Betätigungselement dann wesentlich schneller bewegt als es ohne eine solche Sperre möglich wäre.

Eine erfindungsgemäße Insertionsvorrichtung kann auch wieder verwendbar gestaltet werden, indem in den Insertionsnadelhalter immer wieder neue Insertionsnadeln eingesetzt werden können. Während bei einer disposiblen Vorrichtung zum einmaligen Gebrauch in den Insertionsnadelhalter in der Regel bereits vom Hersteller eine Insertionsnadel eingesetzt wird, können Insertionsvorrichtungen zum mehrmaligen Gebrauch auch ohne in den Insertionsnadelhalter eingesetzte Insertionsnadeln vertrieben werden, da ein Benutzer separat vertriebene Insertionsnadeln selbst in den Insertionsnadelhalter einsetzen kann. Bei einer wieder verwendbaren Insertionsvorrichtung kann es vorteilhaft sein, eine Rückstellfeder vorzusehen, die das oder die Betätigungselemente nach Betätigung zum erneuten Gebrauch in eine Ausgangsposition zurückbewegt. Die mechanischen Anforderungen an eine derartige Rückstellfeder sind außerordentlich gering, so dass eine solche Rückstellfeder kostengünstig, beispielsweise aus Kunststoff, hergestellt werden kann.

Um die quer zur Stichrichtung verlaufende Antriebsbewegung des Betätigungselements in eine lineare Stichbewegung des Insertionsnadelhalters umzusetzen, wird ein Antriebsmechanismus mit einem Pleuel verwendet. Indem die Antriebsbewegung zunächst eine Dreh- oder Schwenkbewegung bewirkt, kann auf diese Weise mit geringerem Aufwand die für eine Stichbewegung erforderliche lineare Bewegung erzeugt werden. Der Antriebsmechanismus weist einen mit dem Betätigungselemente gekoppelten Rotor und einen mit dem Rotor gekoppelten Pleuel auf, der eine Drehbewegung des Rotors in eine lineare Bewegung des Insertionsnadelhalters umsetzt. Eine Kopplung des Betätigungselements mit dem Rotor kann beispielsweise über eine Zahnstange erfolgen.

Prinzipiell genügt bei einer erfindungsgemäßen Insertionsvorrichtung ein einziges Betätigungselement, das quer zur Stichrichtung beweglich ist. Besonders vorteilhaft ist es jedoch, die Insertionsvorrichtung mit zwei gegenüberliegend angeordneten Betätigungselementen auszustatten. Von den Betätigungselementen auf das Gerät bzw. den Antriebsmechanismus ausgeübte Quer- und Reaktionskräfte lassen sich auf diese Weise kompensieren. Zudem kann ein Gerät mit zwei gegenüberliegend angeordneten Betätigungselementen von Rechtshändern ebenso gut wie von Linkshändem verwendet werden. Bevorzugt ist dabei, dass die beiden Betätigungselemente derart mit dem Antriebsmechanismus gekoppelt sind, dass sie sich bei einer Stichbewegung in entgegengesetzter Richtung bewegen. Beispielsweise können sich die beiden Betätigungselemente bei einer Stichbewegung aufeinander zu bewegen. Dies ermöglicht es, die für eine Stichbewegung erforderliche Kraft manuell beispielsweise dadurch aufzubringen, indem das Gerät mit einer Hand umfasst und die Hand zusammengedrückt wird. Eine solche Bewegung entspricht dem Ballen einer Faust, ist also eine sehr einfache Bewegung, die keine besondere Präzision oder Koordination erfordert. Derartige Bewegungen können zudem ausgeführt werden, ohne dass eine Reaktionskraft auf den Körper des Patienten übertragen wird. Die Gefahr eines Verkippens des Geräts während des Insertionsvorgangs lässt sich deshalb wesentlich reduzieren.

Insertierbare Sensoren werden üblicherweise zusammen mit einer Sensorträgereinheit verwendet, die einem Patienten auf den Bauch geklebt werden kann. Zur Insertion eines Sensors kann eine Insertionsvorrichtung mit einer solchen Sensorträgereinheit gekoppelt werden. Nach beendeter Insertion wird die Insertionsvorrichtung üblicherweise von der Sensorträgereinheit gelöst, die am Körper des Patienten verbleibt. Klebende Sensorträgereinheiten mit einer dazu passenden Insertionsvorrichtung sind beispielsweise aus der US 2006/0183984 A1 bekannt.

Eine vorteilhafte Weiterbildung der vorliegenden Erfindung sieht deshalb vor, dass die Insertionsvorrichtung einen Kopplungsmechanismus trägt, der bei einer Insertion eine Sensorträgereinheit an der Insertionsvorrichtung hält und am Ende eines Insertionsvorgangs von dem Antriebsmechanismus betätigt wird, so dass sich die Sensorträgereinheit von der Insertionsvorrichtung löst. Dabei kann es vorteilhaft sein, zusätzlich zu dieser automatischen Abkopplung nach abgeschlossener Insertion eine vorzeitige Abkopplung zu ermöglichen, beispielsweise um eine schmerzhafte Insertion abzubrechen. Hierfür kann der Kopplungsmechanismus mit einem Betätigungselement versehen werden, das ein Benutzer ohne Einwirkung des Antriebsmechanismus betätigen kann.

Bevorzugt hat der Kopplungsmechanismus zwei gelenkig miteinander verbundene Arme, welche die Sensorträgereinheit halten und von dem Antriebsmechanismus bewegt werden, um die Insertionsvorrichtung von der Sensorträgereinheit abzukoppeln.

Weitere Einzelheiten und Vorteile der Erfindung werden an Ausführungsbeispielen unter Bezugnahme auf die beigefügten Zeichnungen erläutert. Gleiche und einander entsprechende Bauteile sind dabei mit übereinstimmenden Bezugszahlen bezeichnet. Es zeigen:
- Figur 1:: ein Ausführungsbeispiel einer erfindungsgemäßen Insertionsvorrichtung bei geöffnetem Gehäuse;
- Figur 2:: ein weiteres Ausführungsbeispiel einer erfindungsgemäßen Insertions- vorrichtung bei geöffnetem Gehäuse;
- Figur 3:: ein weiteres Ausführungsbeispiel einer erfindungsgemäßen Insertions- vorrichtung bei geöffnetem Gehäuse;
- Figur 4:: eine schematische Darstellung der Kopplung einer Insertionsvorrich- tung an eine Sensorträgereinheit;
- Figur 5: eine schematische Darstellung eines weiteren Ausführungsbeispiels der Kopplung einer Insertionsvorrichtung an eine Sensorträgereinheit;
- Figur 6: eine schematische Darstellung eines weiteren Ausführungsbeispiels der Kopplung einer Insertionsvorrichtung an eine Sensorträgereinheit; und
- Figur 7: eine schematische Darstellung eines weiteren Ausführungsbeispiels der Kopplung einer Insertionsvorrichtung an eine Sensorträgereinheit.

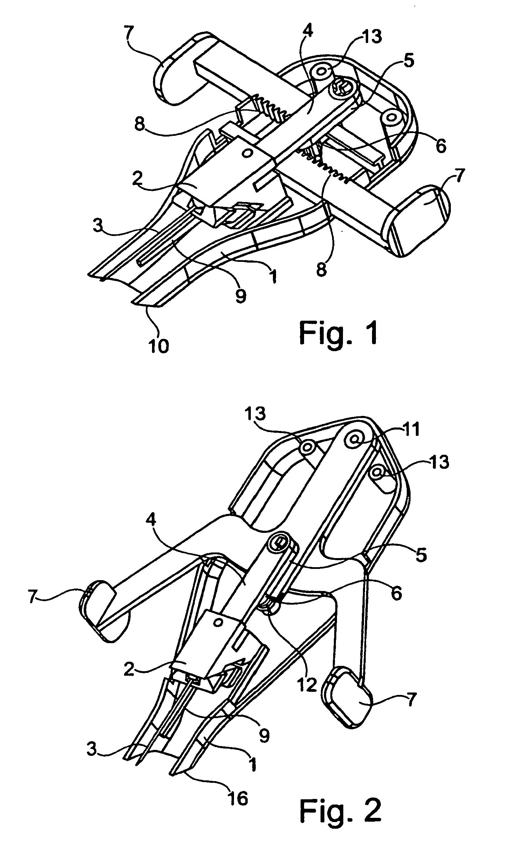

Die in Figur 1 dargestellte Insertionsvorrichtung hat ein Gehäuse 1, in dem ein Insertionsnadelhalter 2 in einer Stichrichtung linear beweglich ist. In Figur 1 ist der Insertionsnadelhalter mit einer eingesetzten Insertionsnadel 3 dargestellt. Der Insertionsnadelhalter 2 ist an einen Antriebsmechanismus gekoppelt, der bei dem dargestellten Ausführungsbeispiel einen Pleuel 4, eine Kurbel 5, einen als Zahnrad ausgebildeten Rotor 6 und zwei Betätigungselemente 7 umfasst. Die Betätigungselemente 7 sind mit Zahnstangen 8 versehen, die mit dem als Zahnrad ausgebildeten Rotor 6 kämmen. Werden die beiden Betätigungselemente 7 in das Gehäuse 1 hineingedrückt, also senkrecht zur Stichrichtung aufeinander zu bewegt, wird der als Zahnrad ausgebildete Rotor 6 in eine Drehbewegung versetzt. Der Rotor 6 ist über eine Kurbel 5 an den Pleuel 4 gekoppelt, der die Drehbewegung des Rotors 6 in eine lineare Bewegung des Insertionsnadelhalters 2 umsetzt. Auf diese Weise erfolgt die Stichbewegung der Insertionsnadel 3 während der Antriebsbewegung der Betätigungselemente 7.

In ihrem in Figur 1 dargestellten Ausgangszustand ragen die beiden Betätigungselemente 7 seitlich aus dem Gehäuse 1 der Insertionsvorrichtung heraus. Insbesondere bei einem Einweggerät, das nach einmaligem Gebrauch bestimmungsgemäß entsorgt wird, können die seitlich aus dem Gehäuse herausragenden Betätigungselemente 7 mit einem Originalitätsschutz, beispielsweise einer Siegelfolie, versehen sein, um dem Benutzer den Neuzustand des Geräts anzuzeigen. Nach dem Entfernen des Originalitätsschutzes kann die Insertionsvorrichtung in die Hand genommen werden, wobei das verbreiterte Gehäuseende im Handballen zum Liegen kommt und die beiden Betätigungselemente 7 von Daumen und Zeigefinger umschlossen werden. Drückt der Benutzer dann die beiden Betätigungselemente 7 gegeneinander, führen diese eine quer zur Stichrichtung verlaufende Antriebsbewegung aus, die von dem beschriebenen Antriebsmechanismus in eine lineare Stichbewegung des Insertionsnadelhalters 2 umgesetzt wird. Um schmerzhafte Querbewegungen der Insertionsnadel 3 zu minimieren, ist eine Linearführung 9 vorgesehen. Der Insertionsnadelhalter 2 ist an die Linearführung 9 gekoppelt, die beispielsweise eine Schiene sein kann, auf oder entlang welcher der Insertionsnadelhalter 2 gleitet.

Vorteilhaft kompensieren sich die von den Betätigungselementen 7 auf den Rotor 8 ausgeübte Querkräfte, so dass reibungserhöhende Ausweichbewegungen des Rotors vermieden werden.

Das Gehäuse 1 hat eine schräg zur Stichrichtung verlaufende Unterseite 10, mit der die Insertionsvorrichtung bei Gebrauch an den Körper eines Patienten angesetzt wird. Bevorzugt verläuft die Unterseite 10 unter einem Winkel von 30° bis 60° zur Stichrichtung, so dass eine Insertionsnadel 3 schräg in das Unterhautfettgewebe eines Benutzers eingestochen werden kann. Durch das Gehäuse 1 ist die Insertionsnadel 3 für einen Benutzer nicht sichtbar, was aus psychologischen Gründen vorteilhaft ist.

Die dargestellte Insertionsvorrichtung dient zum Insertieren eines Sensors für in vivo Messungen, beispielsweise zur Messung der Glukosekonzentration. Der Antriebsmechanismus setzt deshalb die quer zur Stichrichtung verlaufende Antriebsbewegung der Betätigungselemente 7 sowohl in eine Stichbewegung als auch in eine unmittelbar anschließende Rückzugsbewegung des Insertionsnadelhalters 2 um. Dies wird dadurch erreicht, dass die Länge der Zahnstangen 8 der Betätigungselemente 7 genau passend für eine volle 360°-Drehung des Rotors 6 sind. Im vorderen Wendepunkt der Bewegung des Insertionsnadelhalters 2 wird ein an die Insertionsnadel 3 angekoppelter Sensor von der Insertionsnadel 3 getrennt und bleibt somit bei der Rückzugsbewegung in der erzeugten Stichwunde im Körper des Patienten. Die Insertion eines Sensors erfolgt auf diese Weise in dem durchgängigen Bewegungsablauf. Der Patient nimmt lediglich einen Ausgangszustand beim Ansetzten der Insertionsvorrichtung und einen Endzustand nach abgeschlossener Insertion wahr.

Am Ende ihres Betätigungsweges rasten die Betätigungselemente 7 ein, beispielsweise indem sie im oder mit dem Gehäuse 1 verrasten. Auf diese Weise wird der Antriebsmechanismus verriegelt, so dass die Insertionsvorrichtung nur für einen einmaligen Gebrauch verwendet werden kann. Zudem wird durch das Verriegeln des Antriebsmechanismus erreicht, dass die Insertionsnadel 3 nach Gebrauch im Gehäuse 1 bleibt und der Benutzer somit vor einer Verletzung durch eine gebrauchte Insertionsnadel 3 geschützt ist.

Bei der dargestellten Insertionsvorrichtung wird die für eine Stichbewegung erforderliche Kraft während des Stichs vom Benutzer durch die manuelle Betätigung der Betätigungselemente 7 erzeugt. Vorteilhaft wird deshalb weder eine Antriebsfeder noch ein sonstiger Energiespeicher benötigt. Die dargestellte Insertionsvorrichtung ist somit energiespeicherfrei. Energiespeicherfreie Insertionsvorrichtungen werden auch als manuelle Insertionsvorrichtungen bezeichnet.

Figur 2 zeigt ein weiteres Ausführungsbeispiel einer Insertionsvorrichtung, das sich von dem vorstehend beschriebenen Ausführungsbeispiel nur im Aufbau des Antriebsmechanismus unterscheidet. Während bei dem vorstehend beschriebenen Ausführungsbeispiel die quer zur Stichrichtung verlaufende Antriebsbewegung der Betätigungselemente 7 eine lineare Bewegung ist, führen die Betätigungselemente 7 bei dem in Figur 2 dargestellten Ausführungsbeispiel eine Schwenkbewegung aus. Die Betätigungselemente 7 sind hierfür mit einem Schwenklager 11, beispielsweise auf einem Zapfen 11, schwenkbar in dem Gehäuse 1 gelagert.

Zum Betätigen des Antriebsmechanismus werden die beiden Betätigungselemente 7 ebenso wie bei dem vorstehend beschriebenen Ausführungsbeispiel zusammengedrückt. Die Betätigungselemente 7 sind über einen Faden 12 mit einem als Kurbelwelle ausgebildeten Rotor 6 gekoppelt, so dass die Schwenkbewegung der Betätigungselemente 7 eine Drehbewegung des Rotors 6 bewirkt. Der Rotor 6 ist ebenso wie bei dem vorstehend beschriebenen Ausführungsbeispiel über eine Kurbel 5 mit einem Pleuel 4 gekoppelt, so dass die Drehbewegung des Rotors 6 in eine lineare Stechbewegung des Insertionsnadelhalters 2 umgesetzt wird.

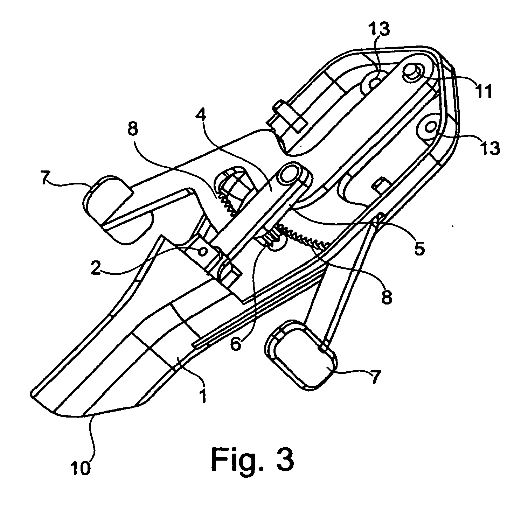

Figur 3 zeigt eine Abwandlung des in Figur 2 dargestellten Ausführungsbeispiels. Bei diesem Ausführungsbeispiel führen die beiden Betätigungselemente 7 ebenso wie bei dem in Figur 2 dargestellten Ausführungsbeispiel eine Schwenkbewegung aus. Ähnlich wie bei dem Ausführungsbeispiel von Figur 1 sind die Betätigungselemente 7 jedoch mit Zahnstangen 8 versehen, die mit einem als Zahnrad ausgebildeten Rotor 6 kämmen. Die Schwenkbewegung der Betätigungselemente 7 um den Lagerzapfen 11 bewirkt deshalb über die gekrümmten Zahnstangen 8 auch bei diesem Ausführungsbeispiel eine Drehbewegung des Rotors 6, die über einen Pleuel 4 in eine lineare Bewegung des Insertionsnadelhalters 2 umgesetzt wird.

Die Gehäuse 1 der Insertionsvorrichtungen können beispielsweise durch Zusammenfügen von zwei Halbschalen gebildet werden. Eine dieser Halbschalen kann hierfür mit Zapfen oder anderen Verbindungselementen versehen werden, die in komplementäre Verbindungselemente 13 der anderen Halbschale eingreifen, beispielsweise in Hohlzapfen oder Buchsen. Möglich ist es auch, das Gehäuse einteilig als zwei über ein Filmscharnier verbundene Hälften auszubilden, die zum Schließen des Gehäuses zusammengeklappt und verrastet oder stoffschlüssig, beispielsweise durch Kleben oder Schweißen verbunden werden. Ein Filmscharnier ist eine Verbindung zwischen zwei Bauteilen, die wegen ihrer reduzierten Materialstärke biegsam ist und so eine Schwenkbewegung der beiden Bauteile zueinander ermöglicht.

Zur Reduktion von Bauteilen lässt sich ein solches Filmscharnier auch an anderen Stellen bei den beschrieben Ausführungsbeispielen einsetzten. Beispielsweise kann der Insertionsnadelhalter 2 über ein Filmscharnier mit dem Pleuel 4 ausgebildet sein. Möglich ist es auch, das Schwenklager 11 des in Figur 2 gezeigten Ausführungsbeispiels durch ein Filmscharnier zu ersetzen.

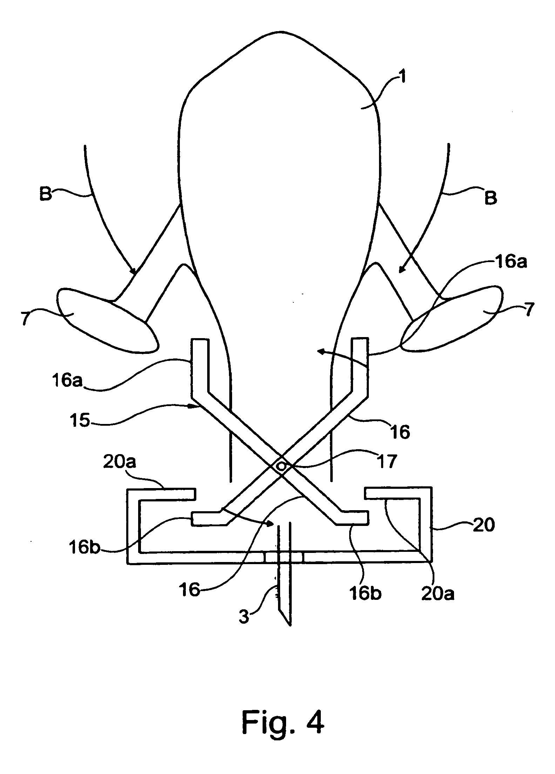

Figur 4 zeigt in einer schematischen Darstellung ein Ausführungsbeispiel einer Sensorträgereinheit 20, die einem Patienten zur Insertion eines Sensors auf den Bauch geklebt werden kann, zusammen mit einer daran angekoppelten Insertionsvorrichtung, wie sie vorstehend beispielsweise unter Bezugnahme auf die Figuren 2 oder 3 beschrieben wurde.

Bei dem in Figur 4 dargestellten Ausführungsbeispiel trägt die Insertionsvorrichtung einen Kopplungsmechanismus 15, über den die Insertionsvorrichtung für eine Insertion an die Sensorträgereinheit 20 gekoppelt ist. Der schematisch dargestellte Kopplungsmechanismus 15 ist als Scherenhebel ausgebildet. Hierzu sind zwei Hebelarme 16 überkreuz angeordnet und um einen Verbindungspunkt 17 schwenkbar. In der in Figur 4 dargestellten Kopplungsposition greifen untere Enden 16b der Hebelarme 16 hinter Halteelemente 20a der Sensorträgereinheit 20 und bewirken so eine mechanische Verbindung zwischen der Insertionsvorrichtung und der Sensorträgereinheit 20.

Bei einer Insertion werden die beiden Betätigungselemente 7 der Insertionsvorrichtung bogenförmig entlang der in Figur 4 dargestellten Pfeile B bewegt, wie dies im Zusammenhang mit den Figuren 2 und 3 erläutert wurde. Am Ende dieser Bewegung treffen die Betätigungselemente 7 auf die oberen Enden 16a der Hebelarme 16 des Kopplungsmechanismus 15. Dadurch werden die oberen Enden 16a der Hebelarme 16 zusammen gedrückt, so dass sich auch deren untere Enden 16b aufeinander zu bewegen und sich dabei aus ihrem Eingriff mit der Sensorträgereinheit 20 lösen. Auf diese Weise entkoppelt die dargestellte Insertionsvorrichtung am Ende eines Insertionsvorgangs automatisch von der Sensorträgereinheit 20 und kann problemlos von ihr abgenommen werden. Die oberen Enden 16a der Hebelarme 16 bilden also Betätigungselemente des Kopplungsmechanismus.

Ein Benutzer hat zudem die Möglichkeit, die Insertionsvorrichtung von der Sensorträgereinheit zu lösen, indem er direkt mit seinen Fingern auf die oberen Enden 16a der Hebelarme 16 drückt und so den Scherenhebelmechanismus betätigt. Auf diese Weise hat ein Benutzer die Möglichkeit, einen Insertionsvorgang abzubrechen und die Insertionsvorrichtung hierfür von Sensorträgereinheit zu lösen.

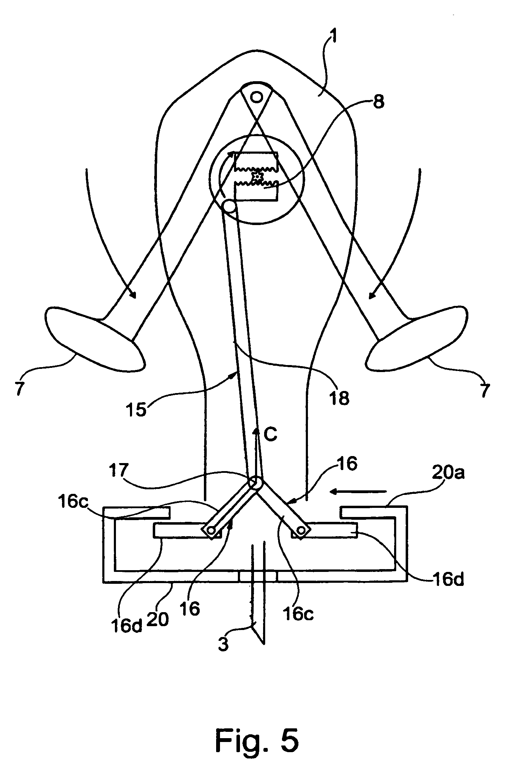

Figur 5 zeigt schematisch ein weiteres Ausführungsbeispiel eines Kopplungsmechanismus 15, der die Insertionsvorrichtung für eine Insertion mit einer Sensorträgereinheit 20 verbindet und nach abgeschlossener Insertion entkoppelt.

Der in Figur 5 schematisch dargestellte Kopplungsmechanismus 15 hat zwei Arme 16, die an einem Verbindungspunkt 17 gelenkig mit einander verbunden sind. Jeder der Arme 16 weist einen Oberarm 16c und einen Unterarm 16d auf, die wie ein Ellenbogen gelenkig mit einander verbunden sind. Die Unterarme 16d greifen in der Figur 5 dargestellten Kopplungsposition in Haltelemente 20a der Sensorträgereinheit 20 ein und bewirken so eine mechanische Verbindung zwischen der Insertionsvorrichtung und der Sensorträgereinheit 20.

An Ende eines Insertionsvorgangs wird der Koppelungsmechanismus 15 ähnlich wie bei dem vorstehend beschriebenen Ausführungsbeispiel von dem Antriebsmechanismus der Insertionsvorrichtung betätigt, so dass die beiden Gelenkarme 16 des Kopplungsmechanismus 15 an ihrem Verbindungspunkt 17 in Richtung des Pfeils C zurückgezogen werden. Dabei bewegen sich die Unterarme 16d aufeinander zu, so dass sich der Kopplungsmechanismus 15 von der Sensoreinheit 20 löst.

Bei dem in Figur 5 dargestellten Ausführungsbeispiel umfasst der Kopplungsmechanismus 15 ein Sinus-Kurbelgetriebe mit einem Arm 18, der als Pleuel gelenkig mit dem Verbindungspunkt 17 der beiden Arme 16 verbunden ist. Das Sinus-Kurbelgetriebe wird über Zahnstangen 8 des Antriebsmechanismus angetrieben, so dass die Sensorträgereinheit 20 am Ende eines Insertionsvorgangs von der Insertionsvorrichtung abgekoppelt wird.

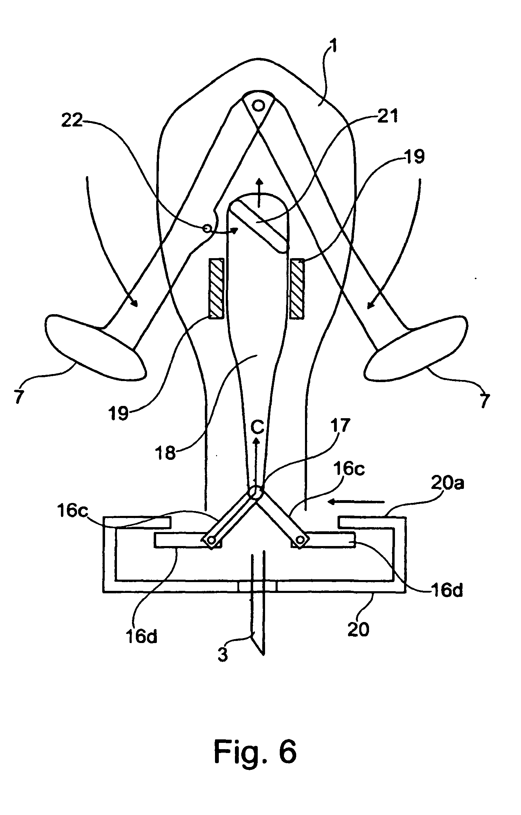

Figur 6 zeigt schematisch eine Variation des in Figur 5 dargestellten Ausführungsbeispiels. Anstelle eines Sinus-Kurbelgetriebes werden die beiden Arme 16 von einem zwischen zwei Linearführungen 19 beweglichen Arm 18 zurückgezogen. Der Arm 18 trägt eine Kulisse 21, die am Ende einer Betätigungsbewegung von einem Zapfen 22 abgefahren wird. Bei dem dargestellten Ausführungsbeispiel ist der Zapfen 22 an einem der beiden Betätigungselemente 7 befestigt und trifft am Ende einer Betätigungsbewegung auf eine Schrägfläche der Kulisse 21. Dies bewirkt eine Bewegung des Arms 18 in Richtung des Pfeils C und so ein Abkoppeln der Insertionsvorrichtung von der Sensorträgereinheit 20.

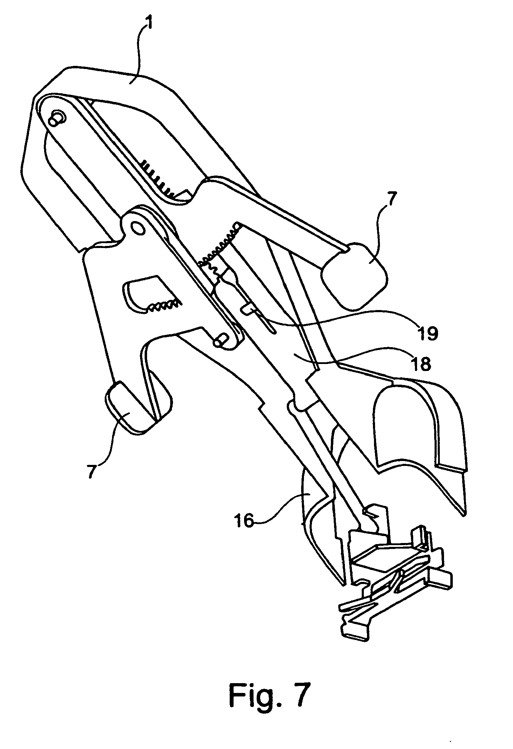

Figur 7 zeigt ein weiteres Ausführungsbeispiel, das eine Abwandlung des vorstehend unter Bezugnahme auf Figur 6 erläuterten Kopplungsmechanismus 15 darstellt. Bei dem in Figur 7 dargestellten Kopplungsmechanismus 15 ist die Linearführung 19 als ein an dem Gehäuse 1 befestigter Zapfen ausgebildet, der in einen Schlitz des Arms 18 eingreift. An dem Arm 18 ist gelenkig ein einziger Arm 16 angebracht, der einen Riegel trägt, der für eine Insertion in die Halteelemente 20a der Sensorträgereinheit 20 eingreift und am Ende eines Insertionsvorgangs von dem Arm 18 aus seiner Eingriffsposition herausgezogen wird, so dass sich die Sensorträgereinheit 20 von der Insertionsvorrichtung 1 löst.

### Bezugszahlen

- 1: Gehäuse
- 2: Insertionsnadelhalter
- 3: Insertionsnadel
- 4: Pleuel
- 5: Kurbel
- 6: Rotor
- 7: Betätigungselement
- 8: Zahnstange
- 9: Linearführung
- 10: Gehäuseunterseite
- 11: Schwenklager
- 12: Faden
- 13: Verbindungselement

- 15: Kopplungsmechanismus
- 16: Arm
- 16a: oberes Ende des Arms
- 16b: unteres Ende des Arms
- 16c: Oberarm
- 16d: Unterarm
- 17: Verbindungspunkt
- 18: Arm
- 19: Linearführung
- 20: Sensorträgereinheit
- 20a: Halteelement
- 21: Kulisse
- 22: Zapfen

## Patentansprüche

1. Insertionsvorrichtung mit
einem Insertionsnadelhalter (2),
einem Antriebsmechanismus (4, 5, 6, 12), um den Insertionsnadelhalter (2) in einer Stichrichtung linear zu bewegen, und
wenigstens einem Betätigungselement (7) zum Betätigen des Antriebsmechanismus (4, 5, 6, 12), wobei
der Antriebsmechanismus (4, 5, 6, 12) eine quer oder entgegengesetzt zur Stichrichtung verlaufende Antriebsbewegung des Betätigungselements (7) in eine Stichbewegung des Insertionsnadelhalters (2) umsetzt,
**dadurch gekennzeichnet, dass** der Antriebsmechanismus (4, 5, 6, 12) einen Rotor (6) und einen mit dem Rotor (6) gekoppelten Pleuel (4) aufweist, der eine Drehbewegung des Rotors (6) in eine lineare Bewegung des Insertionsnadelhalters (2) umsetzt.

2. Insertionsvorrichtung nach Anspruch 1, **dadurch gekennzeichnet, dass** die Stichbewegung während der Antriebsbewegung erfolgt.

3. Insertionsvorrichtung nach einem der vorstehenden Ansprüche, **dadurch gekennzeichnet, dass** der Antriebsmechanismus (4, 5, 6, 12) energiespeicherfrei ist.

4. Insertionsvorrichtung nach einem der vorstehenden Ansprüche, **dadurch gekennzeichnet, dass** der Antriebsmechanismus (4, 5, 6, 12) die Antriebsbewegung des Betätigungselements (7) in eine Drehbewegung des Rotors (6) umsetzt.

5. Insertionsvorrichtung nach einem der vorstehenden Ansprüche, **dadurch gekennzeichnet, dass** die Antriebsbewegung des Betätigungselements (7) eine Schwenkbewegung ist.

6. Insertionsvorrichtung nach einem der vorstehenden Ansprüche, **dadurch gekennzeichnet, dass** das Betätigungselement (7) eine Zahnstange (8) aufweist.

7. Insertionsvorrichtung nach einem der vorstehenden Ansprüche, **dadurch gekennzeichnet, dass** der Antriebsmechanismus (4, 5, 6, 12) in einem Gehäuse (1) angeordnet ist, aus dem das Betätigungselement (7) herausragt.

8. Insertionsvorrichtung nach einem der vorstehenden Ansprüche, **dadurch gekennzeichnet, dass** zwei Betätigungselemente (7) gegenüberliegend angeordnet sind.

9. Insertionsvorrichtung nach einem der vorstehenden Ansprüche, **dadurch gekennzeichnet, dass** sich die beiden Betätigungselemente (7) bei einer Stichbewegung in entgegen gesetzter Richtung bewegen.

10. Insertionsvorrichtung nach einem der vorstehenden Ansprüche, **dadurch gekennzeichnet, dass** sich die beiden Betätigungselemente (7) bei einer Stichbewegung aufeinander zu bewegen.

11. Insertionsvorrichtung nach einem der vorstehenden Ansprüche, **dadurch gekennzeichnet, dass** der Antriebsmechanismus (4, 5, 6, 12) eine quer zur Stichrichtung verlaufende Antriebsbewegung des Betätigungselements (7) in eine Stichbewegung und eine unmittelbar anschließende Rückzugsbewegung des Insertionsnadelhalters (2) umsetzt.

12. Insertionsvorrichtung nach einem der vorstehenden Ansprüche, **gekennzeichnet durch** einer Linearführung (9) für den Insertionsnadelhalter (2).

13. Insertionssystem mit einer Insertionsvorrichtung nach einem der vorstehenden Ansprüche und einer Sensorträgereinheit (20), **dadurch gekennzeichnet, dass** Insertionsvorrichtung einen Kopplungsmechanismus trägt, der bei einer Insertion die Sensorträgereinheit (20) an der Insertionsvorrichtung hält und am Ende eines Insertionsvorgangs von dem Antriebsmechanismus (4, 5, 6, 12) betätigt wird, so dass sich die Sensorträgereinheit (20) von der Insertionsvorrichtung löst.

## Claims

1. An insertion device comprising
an insertion needle holder (2),
a drive mechanism (4, 5, 6, 12) for linearly moving the insertion needle holder (2) in a puncturing direction, and
at least one actuating element (7) for actuating a drive mechanism (4, 5, 6, 12), wherein
the drive mechanism (4, 5, 6, 12) converts a driving motion of the actuating element (7), the driving motion proceeding transversely or opposite to the puncturing direction, into a puncturing motion of the insertion needle holder (2),
**characterized in that** the drive mechanism (4, 5, 6, 12) includes a rotor (6) and a connecting rod (4) coupled to the rotor (6), the connecting rod converting rotary motion of the rotor (6) into linear motion of the insertion needle holder (2).

2. The insertion device according to claim 1, **characterized in that** the puncturing motion occurs during the driving motion.

3. The insertion device according to one of the preceding claims, **characterized in that** the drive mechanism (4, 5, 6, 12) does not include an energy accumulator.

4. The insertion device according to any one of the preceding claims, **characterized in that** the drive mechanism (4, 5, 6, 12) converts the driving motion of the actuating element (7) into rotary motion of the rotor (6).

5. The insertion device according to any one of the preceding claims, **characterized in that** the drive motion of the actuating element (7) is a swivelling motion.

6. The insertion device according to any one of the preceding claims,
**characterized in that** the actuating element (7) includes a gear rod (8).

7. The insertion device according to any one of the preceding claims,
**characterized in that** the drive mechanism (4, 5, 6, 12) is disposed in a housing (1), out of which the actuating element (7) protrudes.

8. The insertion device according to any one of the preceding claims,
**characterized in that** two actuating elements (7) are diametrically opposed.

9. The insertion device according to any one of the preceding claims,
**characterized in that** the two actuating elements (7) move in the opposite direction when a puncturing motion occurs.

10. The insertion device according to any one of the preceding claims,
**characterized in that** the two actuating elements (7) move toward one another when a puncturing motion occurs.

11. The insertion device according to any one of the preceding claims,
**characterized in that** the drive mechanism (4, 5, 6, 12) converts a driving motion of the actuating element (7), the driving motion proceeding transversely to the puncturing direction, into a puncturing motion and, immediately thereafter, into a retraction motion of the insertion needle holder (2).

12. The insertion device according to any one of the preceding claims,
**characterized by** a linear guide (9) for the insertion needle holder (2).

13. An insertion system comprising an insertion device according to any one of the preceding claims, and a sensor carrier unit (20), **characterized in that** the insertion device comprises a coupling mechanism that, for insertion, holds the sensor carrier unit (20) against the insertion device and is actuated by the drive mechanism (4, 5, 6, 12) at the end of an insertion procedure to detach the sensor carrier unit (20) from the insertion device.

## Revendications

1. Dispositif d'insertion comprenant.
un support d'aiguille d'insertion (2),
un mécanisme d'entraînement (4, 5, 6, 12) pour déplacer linéairement le support d'aiguille d'insertion (2) dans un sens de piqûre, et
au moins un élément d'actionnement (7) pour l'actionnement du mécanisme d'entraînement (4, 5, 6, 12);
le mécanisme d'entraînement (4, 5, 6, 12) convertissant un mouvement d'entraînement de l'élément d'actionnement (7), effectué dans le sens transversal ou opposé au sens de piqûre, en un mouvement de piqûre du support d'aiguille d'insertion (2),
**caractérisé en ce que** le mécanisme d'entraînement (4, 5, 6, 12) présente un rotor (6) et une bielle (4) couplée au rotor (6), laquelle convertit un mouvement de rotation du rotor (6) en un mouvement linéaire du support d'aiguille d'insertion (2).

2. Dispositif d'insertion selon la revendication 1, **caractérisé en ce que** le mouvement de piqûre s'effectue pendant le mouvement d'entraînement.

3. Dispositif d'insertion selon l'une quelconque des revendications précédentes, **caractérisé en ce que** le mécanisme d'entraînement (4, 5, 6, 12) est dépourvu d'accumulateur d'énergie.

4. Dispositif d'insertion selon l'une quelconque des revendications précédentes, **caractérisé en ce que** le mécanisme d'entraînement (4, 5, 6, 12) convertit le mouvement d'entraînement de l'élément d'actionnement (7) en un mouvement de rotation du rotor (6).

5. Dispositif d'insertion selon l'une quelconque des revendications précédentes, **caractérisé en ce que** le mouvement d'entraînement de l'élément d'actionnement (7) est un mouvement de basculement.

6. Dispositif d'insertion selon l'une quelconque des revendications précédentes, **caractérisé en ce que** l'élément d'actionnement (7) présente une crémaillère (8).

7. Dispositif d'insertion selon l'une quelconque des revendications précédentes, **caractérisé en ce que** le mécanisme d'entraînement (4, 5, 6, 12) est disposé dans un boîtier (1) duquel dépasse l'élément d'actionnement (7).

8. Dispositif d'insertion selon l'une quelconque des revendications précédentes, **caractérisé en ce que** deux éléments d'actionnement (7) sont disposés l'un en face de l'autre.

9. Dispositif d'insertion selon l'une quelconque des revendications précédentes, **caractérisé en ce que** les deux éléments d'actionnement (7) se déplacent dans une direction opposée lors d'un mouvement de piqûre.

10. Dispositif d'insertion selon l'une quelconque des revendications précédentes, **caractérisé en ce que** les deux éléments d'actionnement (7) se déplacent l'un vers l'autre lors d'un mouvement de piqûre.

11. Dispositif d'insertion selon l'une quelconque des revendications précédentes, **caractérisé en ce que** le mécanisme d'entraînement (4, 5, 6, 12) convertit un mouvement d'entraînement de l'élément d'actionnement (7), effectué transversalement au sens de piqûre, en un mouvement de piqûre et un mouvement de recul directement consécutif du support d'aiguille d'insertion (2).

12. Dispositif d'insertion selon l'une quelconque des revendications précédentes, **caractérisé par** un guide linéaire (9) pour le support d'aiguille d'insertion (2).

13. Système d'insertion doté d'un dispositif d'insertion selon l'une quelconque des revendications précédentes et d'une unité porte-capteur (20), **caractérisé en ce que** le dispositif d'insertion porte un mécanisme d'accouplement qui, lors d'une insertion, maintient l'unité porte-capteur (20) sur le dispositif d'insertion et est actionné à la fin d'un processus d'insertion par le mécanisme d'entraînement (4, 5, 6, 12), de sorte que l'unité porte-capteur (20) se détache du dispositif d'insertion.
